# FASCICULE DE BREVET EUROPEEN

(11) **EP 2 005 181 B1**
(45) Date de publication et mention de la délivrance du brevet: **09.01.2019**
(21) Numéro de dépôt: 07731235.3
(22) Date de dépôt: 30.03.2007
(51) Int. Cl.: G01N 33/564, G01N 33/96

(54) **STANDARD C4D / C4B POUR CYTOMETRIE DE FLUX QUANTITATIVE DU REJET HUMORAL DE TRANSPLANTATION**
C4D/C4B-STANDARD FÜR DIE QUANTITATIVE DURCHFLUSSZYTOMETRIE HUMORALER TRANSPLANTATABSTOSSUNG
C4D/C4B STANDARD FOR QUANTITATIVE FLOW CYTOMETRY OF HUMORAL TRANSPLANT REJECTION

(30) Priorité: 31.03.2006 FR 0602834
(43) Date de publication de la demande: 24.12.2008
(73) Titulaire: Université De Reims Champagne-Ardenne, 51097 Reims Cedex (FR)
(72) Inventeur: COHEN, Jacques, Henri, Max, F-51100 Reims (FR); REVEIL, Brigitte, F-51100 Reims (FR); KISSERLI, Aymric, F-51100 Reims (FR); HAIDAR, Fadi, F-93160 Noisy le Grand (FR); DONVITO, Béatrice, F-51140 Muizon (FR)
(74) Mandataire: Desaix, Anne
(86) Numéro de dépôt international: PCT/FR2007/000557
(87) Numéro de publication internationale: WO 2007/118983

(56) Documents cités:
- WO-A-79/00796
- US-A1- 2005 037 441
- WAHRMANN M. ET AL.: "Flow cytometry based detection of HLA alloantibody mediated classical complement activation." J. IMMUNOL. METH., vol. 275, no. 1-2, 1 avril 2003 (2003-04-01), pages 149-160, XP004416759 ISSN: 0022-1759
- WAHRMANN M ET AL: "[C4d]FlowPRA screening - A specific assay for selective detection of complement-activating anti-HLA alloantibodies." HUM. IMMUNOL., vol. 66, no. 5, mai 2005 (2005-05), pages 526-534, XP004915120 ISSN: 0198-8859
- DATABASE BIOSIS [Online] BIOSCIENCES INFORMATION SERVICE, PHILADELPHIA, PA, US; septembre 2002 (2002-09), NICKERSON P. ET AL.: "C4d deposition in protocol biopsies from flow-cytometry crossmatch (FCXM) positive (+ve) or (-ve) renal transplant recipients (RTR)" XP009075078 Database accession no. PREV200200605430 & JOURNAL OF THE AMERICAN SOCIETY OF NEPHROLOGY, vol. 13, no. Program and Abstracts Issue, septembre 2002 (2002-09), pages 567A-568A, MEETING OF THE AMERICAN SOCIETY OF NEPHROLOGY; PHILADELPHIA, PA, USA; OCTOBER 30-NOVEMBER 04, 2002 ISSN: 1046-6673
- MAGRO C.M. ET AL.: "Use of C4d as a diagnostic adjunct in lung allograft biopsies." AM. J. TRANSPLANT., vol. 3, 2003, pages 1143-1154, XP002408253 cité dans la demande
- BLOY C. ET AL.: "Human monoclonal antibody against Rh(D) antigen: partial characterization of the Rh(D) polypeptide from human erythrocytess", BLOOD, vol. 69, no. 5, 1987, pages 1491-1497,

## Description

La demande divulgue un standard interne utilisable pour l'analyse quantitative du risque de rejet humoral (c'est-à-dire vasculaire) de transplantation. Le standard interne est constitué par une composition stable de composant du complément C4d lié à un support.

La demande a aussi pour objet un procédé pour l'analyse *in vitro* du risque de rejet humoral de transplantation d'organe, dans lequel on détermine la quantité de composant du complément C4d fixé sur les érythrocytes contenus dans un échantillon de sang prélevé chez un patient. La détection du risque en cause concerne le risque de rejet humoral précoce ou le risque de rejet humoral tardif selon les définitions habituellement retenues pour qualifier ce risque. L'invention porte plus particulièrement sur un procédé de détection *in vitro* du risque de rejet humoral de transplantation, chez un patient ayant reçu une transplantation d'organe tel que défini en revendications 1 et 6, ainsi qu'un procédé de détection quantitative *in vitro* du composant du complément C4d déposé sur les érythrocytes contenus dans un échantillon de sang prélevé chez un patient tel que défini en revendications 2 à 4 et 6.

La présente demande divulgue en particulier une composition utilisable comme standard interne pour une analyse en cytométrie en flux quantitative, du composant du complément C4d déposé sur les érythrocytes d'un échantillon de sang prélevé chez un patient.

Il est connu de WO 79/00796 A un procédé de préparation d'érythrocytes animaux recouverts de C4, de façon à dégrader cette molécule le moins possible, afin de pouvoir tester des anticorps anti-C4, notamment anti-C4 natifs ou anti-C4b. Les érythrocytes ainsi préparés ne sont pas adaptés pour déterminer une quantité de C4d, puisqu'ils visent à ne pas comprendre de tel fragments. Par ailleurs, WO 79/00796 A ne divulgue pas un procédé de détection in vitro d'un risque de rejet humoral de transplantation, chez un patient ayant reçu une transplantation d'organe, en particulier fondé sur la mesure du composant du complément C4d s'étant préalablement déposé in vivo sur les érythrocytes d'un patient analysé.

Wahrmann M et al. « Flow cytometry based detection of HLA alloantibody mediated classical complement activation » J. Immunol. Meth., vol 275, no. 1-2, 1 avril 2003, pages 149-160 relate la détection par cytométrie de flux d'alloanticorps anti-HLA fixés sur des billes de capture. Ce document concerne un test appliqué à un sérum prélevé chez un patient, permettant de détecter la présence alloanticorps anti-HLA. L'emploi de billes de capture permet de distinguer des alloanticorps fixant le complément d'alloanticorps ne fixant pas le complément. Le test permet en outre de révéler si les alloanticorps anti-HLA fixés sur des billes de capture ont la capacité ou non d'activer le système du complément, via la fixation de C4 sur les alloanticorps anti-HLA fixés sur les billes.

Wahrmann M et al. « [C4d]FlowPRA screening - A specific assay for selective detection of complement-activating anti-HLA alloantibodies » Hum. Immunol., vol 66, no. 5, mai 2005, pages 526-534 décrit également un test permettant de déterminer dans du sérum de patients dans l'attente d'une transplantation la présence d'alloanticorps fixant le complément, via des billes sur lesquelles du C4d viendrait se déposer. Ce document ne décrit pas un procédé de détection in vitro d'un risque de rejet humoral de transplantation chez un patient ayant reçu une transplantation d'organe.

Le diagnostic d'un rejet humoral de transplantation d'organe chez les patients ayant subi une transplantation reste souvent difficile, en dépit des moyens disponibles, en particulier des moyens invasifs tels que la biopsie de l'organe en question. Cette difficulté peut être liée au fait que des causes diverses peuvent être à l'origine des disfonctionnements observés au niveau de l'organe transplanté : infections, complications chirurgicales, déficiences fonctionnelles tubulo-intersticielles aiguës, récurrence de la maladie initiale et toxicité médicamenteuse. La contribution fréquente de plusieurs causes parmi celles qui viennent d'être citées rend le diagnostic du rejet humoral d'autant plus difficile.

Dans le but de disposer de moyens fiables de diagnostic, réalisés sur des biopsies d'organes transplantés, on s'est intéressé dans le passé à la détection des dépôts de composant du complément C4d dont on a observé qu'ils présentaient un profil linéaire autour des capillaires péri-tubulaires dans les cas de rejet humoral aigu, même en l'absence d'infiltration cellulaire, de dommages vasculaires ou de dépôts d'autres fragments du complément (Habicht 2002). Cette observation a été faite dans le cas de transplantations rénales (Regele H. 2002) mais aussi au niveau d'autres organes transplantés (Magro 2003, Duong Van Huyen 2004, Miller 2004).

La détection de C4d déposé au niveau des organes transplantés dans le cadre d'un test de diagnostic a donc été préconisée, bien qu'il apparaisse difficile de lire les biopsies dans certaines situations complexes, ce qui réduit la valeur clinique de l'observation.

A l'occasion d'une recherche des récepteurs du complément et des fragments du complément présents sur des érythrocytes du sang de patients transplantés rénaux, des dépôts isolés de C4d sur les érythrocytes ont été observés dans différents cas de rejets vasculaires aigus, et ces dépôts étaient également corrélés avec une coloration du C4d dans le rein et chez certains patients présentant des lésions histologiques compatibles avec la survenue d'un rejet humoral expliquant l'échec de la greffe. Réciproquement, aucun accroissement de dépôt de C4d n'a été observé sur des érythrocytes d'organes transplantés ayant connu une suite favorable, ni dans les cas d'échecs aigus de la transplantation corrélés à des biopsies négatives pour la présence de dépôts de C4d. Le même antigène a été retrouvé dans les capillaires d'autres organes transplantés, coeur et foie en particulier, dans la même situation (3, 4, 5). Le C4d a même pu être détecté dans certains cas en l'absence de signe histologique conventionnel de rejet. Initialement décrite dans des cas de rejet précoces, la présence de C4d a aussi pu être démontrée dans des situations de rejets beaucoup plus tardifs, éventuellement intriqués avec des inflitrats suggérant un rejet cellulaire associé. La détection de C4d sur les érythrocytes apparaît donc comme un test sensible et non invasif pour la détermination d'un rejet humoral de greffe.

Le mécanisme du dépôt de C4d n'est pas complètement élucidé : bien qu'il soit corrélé à la présence d'anticorps anti-HLA anti-transplants dans certaines études (Ionescu 2005, Weinstein 2005), l'activation de la voie directe du complément à travers l'activation du complément dépendante de l'anticorps expliquerait difficilement le dépôt extra vasculaire de C4d ou sa présence sur les érythrocytes constatée par les inventeurs, puisque les érythrocytes sont dépourvus d'expression HLA.

Le C4, comme le C3, est une molécule susceptible de fixation covalente en quelques secondes lorsqu'il est activé. Il peut ensuite être dégradé jusqu'au dernier fragment C4d, restant fixé localement par cette liaison covalente. Contrairement au C3, dont les récepteurs cellulaires pour les différentes fractions sont connus, en particulier CR2 / CD 21 / récepteur de C3d ; il n'y a pas de récepteur connu pour le C4d.

La possibilité d'activation du C4, à l'image de celle du C3, hors d'une activation par les anticorps de la voie classique ou par la voie des lectines liant les mannoses est en fait connue depuis plusieurs dizaines d'années dans un tout autre domaine que la transplantation. En effet, les groupes sanguins *Chido* et *Rodgers* sont des polymorphismes du C4 situés sur le C4d et qui ont été décrits à la surface d'érythrocytes avant que ces polymorphismes ne soient rattachés à la molécule plasmatique C4 (8, 9, 10, 11). Des procédures ont été développées aux fins d'immunotypologie permettant la fixation du C4 autologue sur les érythrocytes par une incubation relativement brève à chaud en milieu à faible force ionique en présence de concentrations élevées de sucrose. Ces procédures permettent la fixation de C4 et de C3 qu'il est possible par digestion de réduire en C4d ou C3d.

Ainsi, le dépôt isolé de C4d sur les érythrocytes a également été observé dans les cas de lupus érythémateux systémique (LES) (Manzi 2004, Liu 2005, Liu 2005) à un degré plus élevé que chez les patients transplantés. Un effet « de voisinage », comme dans le cas de maladies à complexes immuns (Freysdottir 3) a été proposé, de même qu'une disparition des érythrocytes portant le composant du complément C3, ce qui conduirait à la persistance dans le sang, des érythrocytes présentant uniquement un dépôt de C4d à l'exclusion d'un dépôt de C3. Compte tenu de la disponibilité du C3 dans le plasma, de l'efficacité des convertases C3 (C4b2b et C3bBb) et de la présence de nombreux accepteurs hydroxyles ou amines sur les érythrocytes pour lier de façon covalente le C3, ce phénomène apparaît être improbable. L'hypothèse de l'activation de C4, en dehors de sa stabilisation par C2 dans la convertase C3 normale aussi bien que les clivages enzymatiques anormaux conduisant à sa liaison covalente aux érythrocytes et à la dégradation finale en fragments C4d, devrait également être considérée (Chakravarti 1987, Atkinson 1988).

Un niveau variable mais faible de C4d sur les érythrocytes de beaucoup de sujets sains, bien qu'il soit stable chez un individu donné, a aussi été observé, suggérant un phénomène d'activation basal comme pour l'activation de C3.

Bien que le mécanisme du dépôt de fragments C4d ne soit pas complètement élucidé, il n'en demeure pas moins que l'élévation du taux de C4d constatée au niveau des biopsies d'organes transplantés constitue une indication intéressante dans la recherche du risque de rejet humoral.

Compte tenu de l'observation qu'ils ont faite, de la présence de C4d à la surface des érythrocytes et de la constatation d'une présence significativement accrue de C4d à la surface des érythrocytes de patients souffrant d'un rejet aigu lors d'une étude multicentrique de greffe de rein, les inventeurs ont défini des moyens de détection du risque de rejet humoral de transplantation, qui soient non invasifs et susceptibles de fournir un résultat quantitatif, fondés sur la détermination du composant du complément C4d déposé sur les érythrocytes dans le sang de patients ayant subi une transplantation.

Les inventeurs ont ainsi défini des conditions permettant de réaliser un dosage quantitatif en faisant appel à un standard interne, analysé comme l'échantillon prélevé chez le patient. En effet la standardisation inter-essais et inter-laboratoires est indispensable à une utilisation clinique de routine. Elle doit s'affranchir à la fois des variations du marquage de révélation du C4d et du réglage de l'appareil de mesure lorsque le cytomètre en flux est utilisé.

Les moyens ainsi définis permettent en d'autres termes de détecter, chez un patient ayant subi une transplantation d'organe, à différents moments, après ladite transplantation, l'éventualité d'un rejet humoral, précoce ou tardif.

La demande concerne donc un procédé de détection *in vitro* du risque de rejet humoral de transplantation, chez un patient ayant reçu une transplantation d'organe, ledit procédé comprenant la détection quantitative d'un composant du complément C4d, déposé sur les érythrocytes contenus dans un échantillon de sang prélevé chez le patient. L'invention porte plus particulièrement sur un tel procédé tel que défini en revendications 1 et 6.

Le procédé de la présente description ou de l'invention s'applique à la transplantation d'organes vascularisés tels que le coeur, le rein, le poumon, le foie, le pancréas et d'autres organes.

Dans un mode de réalisation du procédé de détection ici décrit ou selon l'invention, on réalise d'une part la mesure quantitative du composant du complément C4d de l'échantillon prélevé chez le patient et d'autre part la quantification d'un standard interne, ledit standard interne étant constitué d'une composition de microparticules ou de cellules, recouvertes de composant du complément C4d. La quantité de C4d recouvrant les microparticules ou les cellules est connue en terme de signal émis lorsque le C4d est marqué comme décrit dans la présente demande.

Selon l'invention, le standard interne est constitué par des cellules qui sont des érythrocytes auxquels sont liés les fragments C4d ou des molécules les contenant. Néanmoins, lorsqu'il est question dans la description qui suit, des érythrocytes de la composition de standard interne, la description divulgue également l'application à d'autres cellules qui remplaceraient ou s'ajouteraient aux érythrocytes.

La détection est quantitative dans la mesure où le procédé en question permet de déterminer, directement ou indirectement, la quantité de composant du complément C4d déposée sur les érythrocytes de l'échantillon sanguin prélevé, par référence à ce même composant C4d mesuré sur le standard interne.

Dans les conditions de réalisation de l'invention, la mesure quantitative, c'est-à-dire la détermination de C4d par référence à un standard interne permet donc de déterminer sur un échantillon de sang, si le dépôt observé de C4d sur les érythrocytes est à un niveau normal ou au contraire témoigne d'un rejet humoral de greffe.

La mesure du composant du complément C4d dans l'échantillon prélevé et dans le standard interne est réalisée au moyen d'un marqueur de ce composant, à savoir une molécule qui présente la capacité de se lier de façon spécifique au composant C4d.

Selon un mode de réalisation particulier de l'invention, la détection quantitative du C4d est rendue possible par le marquage de la molécule présentant une affinité de liaison spécifique pour le composant C4d.

La quantification résulte de la comparaison d'un signal émis par le marqueur de la molécule ayant une affinité pour le C4d avec un étalon, établi au moyen du standard interne décrit dans la présente demande, qui est analysé dans les mêmes conditions et pour le même paramètre que l'échantillon de sang prélevé.

Le composant du complément C4d déposé sur les érythrocytes contenus dans un échantillon de sang est détecté et mesuré sous la forme du fragment C4d résultant de la coupure enzymatique du composant du complément C4b ou est détecté alors qu'il est contenu dans une autre molécule, en particulier dans le fragment C4b. Lorsqu'il est question de la détection quantitative de C4d selon l'invention, il s'agit donc de l'une ou l'autre des formes dès lors que le site de liaison du C4d pour le marqueur est accessible.

Le procédé de détection selon l'invention, puisqu'il est mis en oeuvre sur un échantillon de sang, présente l'intérêt d'être non invasif et de pouvoir être utilisé dans le cadre du suivi de la transplantation à différents moments chez les patients.

Par ailleurs, la mesure réalisée dans l'échantillon de sang permet d'écarter l'éventualité d'une maladie de nature différente qui affecterait le taux de C4d, puisque la mesure du niveau de base, avant transplantation, peut être réalisée sur un échantillon de sang du patient, et être prise en compte dans la quantification du composant du complément C4d après transplantation et être alors comparée au résultat obtenu.

Selon un aspect divulgué dans la présente demande, lorsque le composant du complément C4d est lié à des microparticules, il s'agit en particulier de microparticules de latex, en particulier de microsphères, de taille donnée et homogène dans la composition, sur lesquelles sont greffés

les fragments C4d, en tant que tels, ou sous forme accessible intégrée au composant du complément C4b.

Les microparticules sont par exemple des microsphères de polystyrène.

Le diamètre des microparticules, lorsqu'il s'agit de microsphères, est choisi dans un intervalle d'environ 0,1 à environ 5 µm, par exemple environ 1 à 3,5 µm, notamment 3 µm.

Le greffage sur les microparticules ou sur les érythrocytes, des fragments C4d ou de molécules les contenant tels que les fragments C4b, peut être fait par voie d'activation de la cascade enzymatique de la voie classique du complément, ou d'adsorption passive. Selon un autre mode de réalisation ici divulgué, en particulier de l'invention, la greffe est obtenue au moyen d'une liaison chimique, par exemple une liaison covalente le cas échéant au moyen d'un intermédiaire d'activation, par exemple un anticorps.

Lorsque le couplage est réalisé avec du C4d natif, il peut être obtenu par adsorption, ou par liaison covalente.

Lorsque le C4d greffé est une molécule recombinante codant pour tout ou partie du C4d, dans la mesure où il ne comporte pas de groupement thio-ester protégé, le couplage doit être réalisé par une immobilisation covalente artificielle. Tout protocole d'immobilisation peut être utilisé permettant l'activation de certaines fonctions, par exemple en utilisant des carbodiimides, du glutaraldéhyde, ou d'autres types d'activateurs des fonctions de la molécule.

Le cas échéant, des espaceurs peuvent être également utilisés pour éloigner la molécule greffée de la surface de la microparticule ou des érythrocytes. De tels espaceurs sont par exemple des Ig notamment des IgG humaines.

Selon une variante de réalisation, pour greffer les fragments C4d ou des molécules les contenant sur le support choisi, on aura recours à une première couche moléculaire intermédiaire entre les microparticules ou les érythrocytes et lesdits fragments C4d ou les molécules les contenant (notamment C4b), cette couche moléculaire étant chimiquement et/ou biologiquement réactive, et permettant de fonctionnaliser les microparticules ou les érythrocytes de façon à permettre le couplage à des fragments C4d ou à des molécules les contenant, par exemple via des agents de liaison contenus au niveau des fonctions aminés, carboxyles ou hydroxyles de ces molécules intermédiaires.

Encore selon un autre mode de réalisation, l'immobilisation peut être effectuée en utilisant des systèmes de molécules affines permettant une interaction non covalente entre d'autres molécules. A cet égard, on citera les systèmes avidine-biotine ou streptavidine-biotine ou tout système de molécules affines, y compris des systèmes permettant l'immobilisation indirecte par l'intermédiaire d'un couple antigène/anticorps, de façon à réaliser une immunocapture.

Selon le procédé de détection selon l'invention, la quantification du composant du complément C4d déposé sur les érythrocytes d'un échantillon de sang prélevé chez un patient est réalisée d'une part sur un échantillon prélevé avant la transplantation d'organe, d'autre part sur un échantillon de sang prélevé chez ce même patient après la transplantation d'organe, le cas échéant à différents moments après ladite transplantation pour la réalisation d'un suivi de l'évolution de la greffe.

Dans ces conditions, la quantification du composant du complément C4d peut être effectuée en soustrayant le taux de C4d (niveau de base) présent sur les érythrocytes dans l'échantillon prélevé avant la transplantation, du niveau de C4d déposé sur les érythrocytes mesuré sur l'échantillon de sang prélevé chez ce même patient après la transplantation d'organe. Cette quantité de C4d fixée est ensuite interpolée sur la courbe du standard interne permettant de la convertir en une fraction du standard de référence, de préférence exprimée en unités de ce standard (unités arbitraires) ou en sites antigéniques C4d.

Pour la réalisation du procédé de détection décrit dans la présente demande, on met en oeuvre une molécule présentant une affinité de reconnaissance et de liaison spécifique pour le fragment C4d, cette molécule étant le cas échéant marquée, par exemple par un fluorochrome. Pour que la détection puisse être quantitative, la molécule présentant une affinité de reconnaissance et de liaison spécifique pour C4d doit être mise en oeuvre dans des conditions de saturation ou doit avoir une valence de liaison simple (monovalente).

Par l'expression « liaison spécifique » avec le composant du complément C4d, on entend une molécule apte à reconnaître et à lier ce composant du complément de façon privilégiée par rapport à toute autre liaison avec les composés du milieu réactionnel. Avantageusement, la molécule en question a une affinité de liaison spécifique pour C4d telle, qu'elle ne lie pas significativement d'autres composants du milieu réactionnel.

Comme indiqué plus haut, le composant du complément C4d peut être reconnu comme un fragment en tant que tel, ou peut être reconnu au sein d'une molécule qui le contient, par exemple au sein du fragment du composant du complément C4b.

Pour réaliser les microparticules ou les érythrocytes recouverts de composant du complément C4d, on peut utiliser du sang frais normal ou du sérum frais normal comme source du complément. Lorsque le standard interne est constitué d'érythrocytes, l'activation du complément consiste en une autoactivation du sang frais utilisé. Le sang ou le sérum est traité pour permettre l'activation du complément, de façon totale ou partielle, afin de générer des fragments C4d, le cas échéant contenus pour une partie d'entre eux, dans le fragment C4b. L'activation du complément pour produire le C4d est décrite plus en détail dans les exemples qui suivent.

Selon un mode de réalisation particulier, l'activation du complément est totale, de sorte que les microparticules ou les érythrocytes sont recouverts essentiellement de C4d (par rapport à C4b). Dans ce cas, on traite encore éventuellement les microparticules ou les érythrocytes recouverts de C4b, C4d avec une enzyme protéolytique par exemple la trypsine ou la papaine, de façon à diminuer (par dégradation) la quantité de C4d fixé. De cette façon, et en particulier si on réalise une digestion ménagée par la trypsine ou par une autre enzyme protéolytique, durant par exemple jusqu'à 15 minutes, on aboutit à des quantités de C4d sur les microparticules ou sur les érythrocytes qui sont de l'ordre de celles que l'on observe sur les érythrocytes du sang d'un patient dans un cas de rejet de greffe.

Lorsque le support des fragments C4d ou des molécules les contenant est constitué d'érythrocytes, ces derniers, une fois recouverts de C4d sont stabilisés et conservés sous la forme d'une suspension.

La stabilisation est réalisée par tous moyens appropriés, par exemple dans des milieux sucrés hypertoniques, CPD, ACD, ou du paraformaldéhyde ou du formol.

A titre d'exemple, un milieu permettant la conservation de la suspension d'érythrocytes dans des conditions adéquates de stabilité est un milieu ID-CellStab (Diamed-ID, Cressier s/Morat, Suisse) ou un milieu de composition analogue.

La conservation des suspensions d'érythrocytes ou de microparticules recouverts de C4d peut être réalisée à 4°C.

La quantité de fragments C4d ou de molécules les contenant recouvrant les microparticules ou les érythrocytes est de préférence dans un ordre de grandeur voisin de la quantité de fragments C4d résultant de l'activation du complément dans une situation pathologique correspondant au rejet humoral de transplantation. Selon l'invention, la quantité de C4d fixée sur les érythrocytes est telle que mentionnée dans les revendications 1, 2 et 7.

Le standard interne est avantageusement constitué de plusieurs populations de billes de latex ou d'érythrocytes recouverts de C4d chaque population ayant une densité en C4d donnée, différente de celle des autres populations que l'on met en relation avec un signal de marquage (par exemple un niveau de fluorescence donné) pour réaliser la calibration. Les différentes populations peuvent résulter d'un traitement d'une première population (à forte concentration de C4d) avec une enzyme protéolytique dégradant le C4d.

C'est pourquoi, lorsque l'apport en fragments C4d résulte de l'activation complète du complément dans un échantillon de sang normal, on peut traiter les particules ou les érythrocytes recouverts avec les fragments C4d pour en supprimer une partie. Le traitement par une enzyme telle que la trypsine conduit par exemple à garder les fragments C4d fixés sur des sucres au niveau de la surface des érythrocytes.

Selon l'invention, les fragments C4d déposés ou accessibles au sein de molécules les contenant occupent entre 100 et 5000 voire jusqu'à 10000 sites d'une microparticule ou d'un érythrocyte de la composition constituant le standard interne.

Le standard interne ainsi préparé peut comporter, fixés sur les microparticules ou les érythrocytes également d'autres constituants, par exemple d'autres composants du complément, notamment du C3, sans que la pertinence de la mesure des fragments C4d soit affectée.

Dans le cadre de son utilisation comme standard interne, la composition de microparticules ou d'érythrocytes est marquée avec des molécules ayant une affinité de reconnaissance et de liaison spécifique pour le C4d.

Ce marquage est aussi mis en oeuvre pour analyser les microparticules ou les érythrocytes du standard interne et le cas échéant pour préparer des courbes de calibration.

Selon un mode de réalisation particulier ici décrit ou de l'invention, la molécule présentant une affinité de liaison spécifique pour le C4d (en tant que fragment au sein de molécules le contenant) est un anticorps par exemple un anticorps monoclonal, ou un fragment d'anticorps, ayant une affinité pour C4d dans les conditions de la présente demande.

Dans un mode de réalisation particulier ici décrit ou de l'invention, on utilise des fragments monovalents d'anticorps, par exemple des fragments Fab.

Dans un autre mode de réalisation ici décrit ou de l'invention, on utilise un récepteur du C4d naturel (C4bp) ou artificiel.

Des anticorps ou fragments d'anticorps utilisables pour marquer le C4d sont marqués, par exemple avec un fluorochrome afin de les rendre détectables notamment dans un analyse par cytométrie en flux. Le cas échéant, ces anticorps sont également liés à une molécule agissant comme intermédiaire de liaison avec les microparticules ou les érythrocytes.

Des anticorps appropriés pour réaliser l'invention sont par exemple les anticorps monoclonaux biotinylés anti-C4d de la société Quidel (San Diego, CA, USA).

Un procédé particulier de dosage quantitatif de C4 déposé en tant que tel ou au sein de molécules les contenant, à la surface d'érythrocytes d'un échantillon de sang selon l'invention comprend donc une analyse quantitative en cytométrie en flux. Dans ce cas, les conditions habituelles de l'analyse concernant le temps d'incubation, les quantités d'échantillon et de réactif, de marqueur fluorescents sont respectées ainsi que le contrôle de la stabilité du cytomètre.

Alternativement, il est décrit que ce dosage peut être effectué par un test ELISA comprenant une phase liquide soit après lyse et solubilisation des érythrocytes d'un volume donné de sang, soit en révélant en phase liquide la fixation sur les érythrocytes.

Le présente demande divulgue également un procédé de détection quantitative *in vitro,* par cytométrie en flux, du composant du complément C4d déposé sur les érythrocytes contenus dans un échantillon de sang prélevé chez un patient, comprenant les étapes suivantes :
a) d'une part le marquage, au moyen d'une première molécule associée à un marqueur, par exemple un marqueur fluorescent et ayant la capacité de reconnaître et de lier spécifiquement, le composant du complément C4d déposé sur les érythrocytes dudit échantillon de sang ; et d'autre part le marquage au moyen d'une molécule associée à un marqueur, par exemple fluorescent, et ayant la capacité de reconnaître et de lier spécifiquement, le composant du complément C4d, d'une ou plusieurs compositions d'érythrocytes stabilisés ou de microparticules recouverts de composant du complément C4d ladite (lesdites) compositions constituant un standard interne pour la quantification,
b) l'introduction dans un cytomètre en flux, de l'échantillon à quantifier et de la (les) compositions de standard interne ;
c) la détection du signal, par exemple de la fluorescence, induit par chacun des susdits marqueurs fluorescents spécifiques ;
d) la quantification du signal de fluorescence associé aux composants du complément C4d déposés sur les érythrocytes de l'échantillon testé.

L'invention concerne plus particulièrement un procédé de détection quantitative *in vitro* du composant du complément C4d déposé sur les érythrocytes contenus dans un échantillon de sang prélevé chez un patient tel que défini en revendications 2 à 4 et 6.

Lorsque plusieurs compositions de standard interne sont utilisées, chacune correspond à une population de microparticules ou d'érythrocytes recouverts de C4d comme décrit dans la demande, à une densité donnée de C4d corrélable à un niveau de marquage donné. Chaque composition donne donc une valeur étalon à laquelle on peut comparer la valeur obtenue par le marquage de l'échantillon.

Pour déterminer la valeur de marquage de l'échantillon testé, on peut par exemple la situer par rapport à un standard interne haut et par rapport à un standard interne bas correspondant respectivement à des populations ayant des densités différentes de C4d.

L'échantillon d'une part et chaque composition de standard interne d'autre part, sont analysés dans la même série de mesures effectuées mais séparément les uns des autres puisqu'on mesure le même paramètre (notamment la fluorescence) pour chacun d'eux.

Dans le cadre d'un tel procédé conforme à l'invention, on ajoute une étape qui consiste, à l'issue de l'étape d) ci-dessus, à effectuer une étape de comparaison de la valeur du signal, par exemple de fluorescence obtenu pour l'échantillon, ou du nombre de fragments de C4d présents sur les érythrocytes déduit à partir de la valeur de ce signal, avec des valeurs de signal, par exemple de fluorescence, ou avec le nombre de fragments C4d que l'on peut en déduire, obtenus sur un échantillon de sang du patient prélevé avant la transplantation, ou sur un échantillon normal.

Le résultat de l'analyse de la fluorescence est donné soit sous la forme d'une intensité relative de fluorescence correspondant à l'Intensité de Fluorescence Moyenne (MFC : Mean or mediane fluorescence channel) soit sous la forme d'unités arbitraires de C4d ou de sites antigéniques C4d.

Un ratio échantillon/standard interne peut aussi être exprimé.

Ainsi, selon un mode de réalisation du procédé de l'invention, la mesure de fluorescence est une mesure de l'intensité de fluorescence du canal moyen de fluorescence de chaque population de particules et/ou d'érythrocytes considérée.

On appelle « population » les catégories de particules ou de cellules, plus précisément de microparticules ou d'érythrocytes, considérées d'une part pour l'échantillon de sang testé (dont les érythrocytes forment une première population) et d'autre part, pour les compositions constituant le standard interne, recouvertes de composants de complément C4d (en tant que fragment C4d ou au sein d'une molécule le contenant) ou d'érythrocytes stabilisés recouverts de ce même composant et ayant une densité donnée de ce même composant.

La mesure de fluorescence du canal moyen de fluorescence obtenue peut être corrigée pour en soustraire le bruit de fond de l'analyse, conduisant à une valeur correspondant à la valeur de fluorescence du canal moyen de fluorescence corrigée (CMFC).

Cette valeur est exprimée en unités arbitraires de C4d déterminées sur une courbe de calibration préparée au moyen du standard interne et que l'on rapproche des mesures d'intensité de fluorescence du canal moyen de fluorescence dont il est question ci-dessus.

La mesure de C4d déposé sur les érythrocytes d'un échantillon de sang d'un patient, associée à la même mesure opérée sur un standard interne, permet de s'assurer d'une concordance des mesures effectuées sur un même cytomètre et d'une concordance des mesures inter-laboratoires.

Dans un mode de réalisation particulier de l'invention, le résultat de l'analyse de fluorescence du standard interne est exprimé en nombre de sites C4d sur les microparticules ou sur les érythrocytes. On obtient ce nombre en appliquant la méthode de Scatchard.

Dans ce cas, on peut, par référence au standard interne mesurer le nombre de sites C4d sur les érythrocytes de l'échantillon de sang.

Le procédé décrit à l'aide des étapes ci-dessus définies, est avantageusement utilisé pour la détection du risque de rejet humoral de transplantation, chez un patient ayant reçu une transplantation d'organe, comme défini en revendication 5.

On a vu plus haut que le composant du complément C4d est détecté au moyen d'une molécule ayant la capacité de reconnaître et de lier spécifiquement ledit C4d, ladite molécule étant par exemple constituée par un anticorps ou un fragment d'anticorps conjugué à une molécule fluorescente. La phycoérythrine, le DTAF ou d'autres fluorochromes à rendement linéaire peuvent être utilisés.

La molécule ayant la capacité de reconnaître et de lier spécifiquement et de façon monovalente ledit C4d peut être un anticorps monoclonal anti-C4d ou un fragment d'un tel anticorps.

Des fragments d'anticorps utilisables pour la réalisation de l'invention sont par exemple des fragments Fab ou des sous-fragments de ces derniers, dès lors qu'ils conservent le site de reconnaissance et de liaison à la molécule C4d (constituée par le fragment C4d résultant de l'activation du complément ou par toute molécule le contenant telle que le fragment C4b).

La présente demande divulgue également une suspension stabilisée d'érythrocytes recouverts de composant du complément C4d, ledit composant du complément C4d étant lié par liaison covalente directe ou indirecte, aux érythrocytes.

Différentes modalités de réalisation des érythrocytes recouverts de composants du complément C4d ont été exposées ci-dessus en particulier pour obtenir une liaison covalente, étant entendu que la fonctionnalisation ainsi réalisée des érythrocytes doit permettre l'accès du composant du complément C4d lié aux molécules utilisées pour le détecter et le quantifier.

Comme indiqué plus haut, le composant du complément C4d n'est pas nécessairement la seule molécule présente sur la surface des érythrocytes de la suspension. Ainsi, d'autres composants du complément, par exemple des fragments C3 peuvent également être présents sur les érythrocytes pour peu qu'ils ne gênent pas la mesure quantitative du composant C4d ce qui sera mesuré expérimentalement.

La quantité de composants du complément C4d recouvrant les érythrocytes de la suspension est telle qu'elle avoisine la quantité habituellement présente sur les érythrocytes d'un échantillon de sang caractéristique d'une situation de rejet humoral de transplantation d'organes.

A titre indicatif, on a précisé plus haut des paramètres tenant à la quantité de C4d fixée sur les érythrocytes de la suspension stabilisée.

La suspension stabilisée d'érythrocytes ainsi définie peut être définie en ce qu'elle est obtenue après mise en contact *in vitro* des érythrocytes avec un échantillon de sang ou de plasma frais normal, dans des conditions d'activation contrôlée (y compris par exemple permettant l'activation totale) du complément de façon à produire du C4b dans lequel le C4d est accessible ou des fragments de C4d puis récupération des érythrocytes recouverts de composant du complément C4d ou de molécules le contenant.

Le C4d présent sur les érythrocytes de la suspension réalisée peut être natif, notamment lorsqu'il est obtenu par activation du complément d'un échantillon de sang ou de plasma.

Alternativement, il peut s'agir d'une molécule de C4d recombinante, qui est alors dépourvue de fonction thio-ester protégée. Dans ce cas, la liaison covalente avec les érythrocytes fait intervenir une activation des fonctions chimiques de la molécule susceptibles de participer à la liaison covalente.

On entend par échantillon de sang ou plasma frais normal, un échantillon de sang ou de plasma prélevé chez un patient qui n'a pas subi de transplantation d'organe et de préférence à partir d'un échantillon de sang recueilli sur un anticoagulant inhibant l'activation du complément, par exemple l'EDTA.

Comme décrit plus haut, pour réaliser la suspension stabilisée d'érythrocytes recouverte de composant du complément C4d, on peut avoir recours à toute méthode d'association connue de molécules avec la surface d'une cellule, et on peut notamment procéder par couplage covalent aux érythrocytes. Les méthodes de couplage ont été décrites précédemment sont applicables en particulier à la suspension stabilisée d'érythrocytes selon l'invention.

La demande décrit également l'utilisation d'une suspension stabilisée d'érythrocytes ou de microparticules sur lesquels sont déposés des composants du complément C4d, en tant que standard interne dans un procédé de détection *in vitro* du risque de rejet humoral de transplantation chez un patient.

La demande décrit également un kit utilisable pour la détection du risque de rejet humoral de transplantation, dans lequel sont présentes :
- une ou plusieurs compositions constituant un standard interne de quantification du composant du complément C4d, ladite (lesdites) compositions comprenant des particules ou des érythrocytes stabilisés en suspension, recouverts de composant du complément C4d sous forme du fragment C4d ou d'une molécule le contenant,
- une courbe de calibration pour les compositions de standard interne, permettant de relier les mesures relatives d'intensité de fluorescence, à des unités arbitraires, ladite courbe étant obtenue par une analyse en cytométrie en flux, et le bruit de fond étant le cas échéant soustrait pour obtenir une valeur corrigée,
- le cas échéant, une composition de molécules ayant la capacité de reconnaître et de lier de façon spécifique le composant du complément C4d, lesdites molécules étant de préférence marquées, par exemple par un fluorochrome.

L'invention concerne encore l'utilisation d'un kit tel que défini en revendication 7.

D'autres propriétés caractéristiques de l'invention apparaissent dans les exemples et les figures qui suivent.

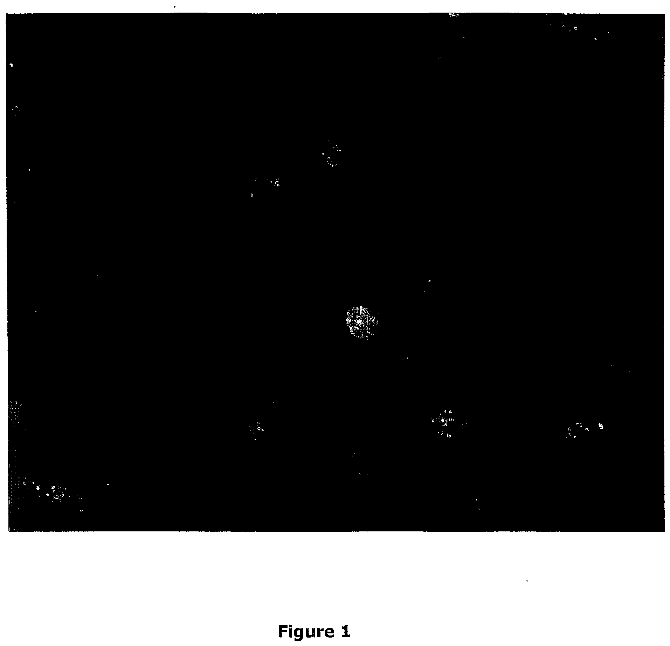

Figure 1 : Analyse d'échantillons. Mise en évidence de C4d déposé sur les érythrocytes d'échantillons de sang.
Figure 2
   -A- Mesure de la fluorescence d'une population d'érythrocytes recouverts de fragments C4b (EC4b) dans lesquels le site de liaison de C4d est accessible donnant un canal moyen de fluorescence de 286,41 (dont 14,97 du contrôle négatif) ;
   -B- Mesure de la fluorescence d'une population d'érythrocytes recouverts de fragments C4d (EC4d) obtenue par traitement avec la trypsine, de la population EC4b donnant un canal moyen de fluorescence de 69,02 (dont 4,97 du contrôle négatif).

Dans les deux cas, les érythrocytes contrôle négatif ont donné un canal moyen de fluorescence de 14,97.

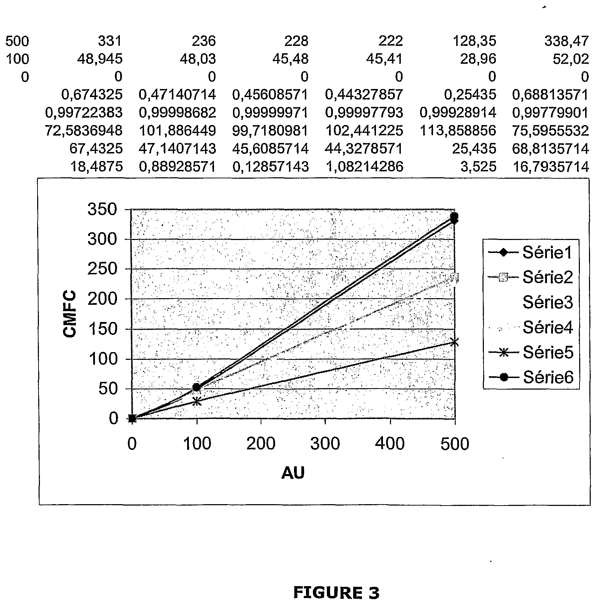

Figure 3 : Courbes de calibration de différentes populations d'érythrocytes recouverts de C4d selon la méthode décrite plus loin.

### Exemples

### I - Protocole d'analyse des échantillons

Des échantillons de sang de patients ayant subi une transplantation rénale ont été prélevés chez des patients sur anti-coagulant EDTA à JO, J8, J30 ou J90 après la greffe ou au moment d'une crise de rejet.

Les analyses effectuées l'ont été dans le respect des règles éthiques et légales, de même qu'en conformité avec la charte d'Helsinki.

La distribution de la coloration de C4d évaluée par cytométrie en flux était très large chez un individu donné, beaucoup d'érythrocytes peu ou pas de C4d (figure 1), suggérant même chez certains patients, un effet de population double. Sur la base d'un examen au microscope par epi-fluorescence, la coloration du C4d sur les érythrocytes s'est en effet avérée également très hétérogène. Cette distribution était en contraste avec la distribution plus homogène de C4 sur les érythrocytes de patients atteints de LES. Globalement, les rejets aigus les plus manifestes montraient le plus fort degré de dépôt de C4d. La quantification de C4d sur les érythrocytes a été testée en utilisant soit les canaux moyens de fluorescence (CMF) sur un cytomètre de flux calibré avec les billes ou exprimant en unité arbitraire, l'interpolation des CMF au moyen d'un standard externe fait à partir de la coloration quantitative d'un autre antigène (CR1) sur le même érythrocyte, révélé en utilisant le même système de coloration. La reproductibilité au jour le jour en utilisant ces méthodes de standardisation était jugée inadéquate. Il est donc apparu très important de mettre au point un standard interne par exemple au moyen d'érythrocytes ou de billes (Spycher 1991) portant du C4d stabilisés afin d'améliorer la reproductibilité permettant à la fois de considérer les variations faibles en C4d durant la période de suivi des patients transplantés et pour la standardisation inter-laboratoires pour un suivi homogène des patients.

### Méthodes

Le sang de patients de deux centres de transplantations rénale a été recueilli sur anticoagulant EDTA au jour J0, J8, J30 ou J90, ou au moment d'une crise de rejet. Des volontaires sains constituant des contrôles négatifs ont été sollicités également.

### Coloration des érythrocytes

Après avoir été lavés trois fois dans un tampon PBS/BSA à 0,15%, les érythrocytes ont été incubés avec un anticorps monoclonal biotinylé anti-C4d (Quidel San Diego, CA, USA) pendant 45 minutes à 4°C. Les anticorps fixés ont été révélés avec un système amélioré Phytoérythrine-Streptavidine (Caltag Lab, Burlingam CA, USA) / antistreptavidine (Vector Lab, Burlingam CA, USA). Lanalyse de cytométrie en flux a été réalisée en utilisant un dispositif linéaire de photomultiplicateur pour éviter toute distorsion de la carte de conversion en log. On a utilisé comme standard externe, un antigène CR1 coloré avec un anticorps monoclonal anti-CR1 biotinylé J3D3 et le même système de révélation, puis quantifié en utilisant une courbe de référence d'érythrocytes ayant une densité en CR1 connue (Cohen 1987). La même courbe a été utilisée comme standard externe pour donner le niveau de C4d sur les érythrocytes, en Unités Arbitraires. Les deux colorations ont été analysées au moyen des cytomètres Facstar+ ou Facscan (Becton Dickinson Mountain View, CA, USA), calibrés en utilisant des billes de calibration fluorescentes (Calibrite, Becton Dickinson, Mountain View, CA, USA).

### Histologie

Une analyse histologique a été effectuée parallèlement pour examiner la corrélation des résultats obtenus avec ceux résultant de l'analyse des érythrocytes. La coloration au Trichome de Masson a été réalisée sur des biopsies de reins. Les dommages survenus sur les tissus et les lésions dues au rejet ont été enregistrés selon le système BANFF (Racusen 1999, Racusen 2003). La coloration des C4d a été réalisée au moyen d'un anticorps monoclonal anti-C4d (Quidel San Diego, CA, USA) et un anticorps de chèvre biotinylé anti-Ig de souris (Amersham Biosciences Orsay, France) puis un système de révélation streptavidine Alexa Fluor 488 (Molecular Probes Eugene, OR, USA).

Les résultats de l'analyse sont rapportés sur la Figure 1.

### II Protocoles de préparation de composition de standard interne

### Préparation de microparticules de latex recouvertes de C4d

La préparation est inspirée de la technique décrite par Spycher et al (Spycher 1991).

Des billes de latex de diamètre de 3µ (L300 Prolabo®) ont été incubées avec 5mg/ml d'IgG agrégées à chaud, pendant une nuit à 4°C dans un tampon phosphate salin. Les Ig agrégées à chaud ont été préalablement préparées en les diluant du Tegeline® à 10mg/ml (LFB, Lille, France) dans un tampon phosphate salin, et en les incubant pendant 30minutes à 60°C.

Les billes de latex recouvertes d'Ig ont été recouvertes de C4d par le biais d'une activation conventionnelle du complément, par la voie classique : les billes recouvertes d'Ig ont été exposées à du sérum frais humain comme source de complément, le sérum étant dilué au 1 :20 dans un tampon véronal (VBS++) avec du Ca 0,15mM, Mg 0,5mM, et 1mM/ml BSA. Le contact du sérum avec les billes est maintenu pendant 30 minutes à 37°C. Puis les billes sont lavées et conservées à 4°C en VBS, EDTA 5mM, 0,2% NaN3, 1mM PMSF .

Les billes de latex ont ensuite été colorées (de la même façon que les érythrocytes des échantillons) pour la quantification en utilisant un anticorps monoclonal anti-C4d biotinylé (ref A704 Quidel San Diego CA, USA) et un système de révélation Phytoérythrine-Streptavidine (Caltag Lab, Burlingam CA, USA) / antistreptavidine (Vector Lab, Burlingam CA, USA).

L'appareil de cytométrie en flux a été réglé pour que l'on obtienne le canal moyen de fluorescence (MFC) des billes colorées dans le canal choisi comme référence pour la standardisation au jour le jour en utilisant ce lot de billes.

### Préparation d'érythrocytes recouverts de C4d

On a préparé *in vitro* des érythrocytes recouverts de C4d par le biais d'une autoactivation de C4 autologue sur des érythrocytes, suivant le protocole décrit ci-dessous :

### Globules rouges

Des échantillons de sang humain recueillis dans des vacutainers contenant 0,12 ml d'EDTA à 0,15 % obtenu chez de volontaires sains ayant donné leur consentement informé conformément à la réglementation française concernant les aspects éthiques de la recherche médicale aussi bien que conformément à la charte d'Elsinki.

### Solution à faible force ionique (LIS)

Une solution à 10% de sucrose contenant des ions Ca++ et Mg++ consistant en 100 g/l de sucrose dans de l'eau distillée avec 0,15 mM Ca++ (sous forme de CaCl₂) et 0,5 mM Mg++ (sous forme de MgCl₂) a été utilisée. Cette solution du sucrose est référencée ci-après « milieu LIS ».

### Recouvrement des globules rouges avec C4b

Une moitié de la quantité de sang frais a été ajoutée à 12,5 ml de milieu LIS et l'ensemble a été mélangé à 37°C pendant 30 minutes. Les globules rouges ont été lavés dans le milieu LIS en soumettant le tube à 2410 g à 4°C pendant 5 minutes. Cette suspension de globules rouges est référencée ci-après « EC4b ». Les cellules ont été conservées à 4°C dans un milieu ID-CellStab (Diamed-ID, Cressier s/ Morat, Suisse).

8µl de C4b ont été lavées dans 3 ml de tampon TBS-BSA (0,12 %) (Biomérieux, Marcy L'Etoile, France- SIGMA, Lyon, France) en vue de l'immunocoloration avec des anti-C4d.

### Traitement à la trypsine (ou une autre enzyme protéolytique)

Le traitement avec une enzyme protéolytique permet de dégrader une partie du C4 fixé, permettant de disposer d'érythrocytes recouverts de quantités différentes de C4d.

Un volume d'une suspension à 30% de cellules EC4b lavées a été mélangé avec un volume égal de trypsine-EDTA à 0,1% (Gibco, Cergy Pontoise, France) et incubé à 37°C pendant 15 minutes sous agitation douce.

Les érythrocytes obtenus comportent alors une concentration en C4d de l'ordre de celle que l'on rencontre lors du rejet humoral de greffe, sur les échantillons de sang d'un patient.

Ces cellules ont été lavées dans un milieu LIS en soumettant les tubes à 2410 g, à 4°C pendant 5 minutes. Cette suspension de globules rouges est référencée ci-après comme : « EC4d ». Les cellules ont été conservées à 4°C dans un milieu ID-CellStab (Diamed-ID, Cressier s/ Morat, Suisse).

8 µl de EC4d ont été lavées dans 3 ml de tampon PBS/BSA (0,12 %) en vue de la coloration avec un anticorps anti-C4d.

L'objectif était d'obtenir des densités de C4d comparables à celles correspondant aux marquages observés en clinique et par ailleurs, une stabilité dans le temps suffisante pour l'utilisation de la composition comme standard interne de calibration.

### Coloration anti C4d

La coloration anti C4d a été réalisée en utilisant un anticorps monoclonal anti C4d et un système amplifié antistreptavidine biotinylé - streptavidine Alexa 488, de la même façon que les érythrocytes de patients ont été colorés. Puis l'analyse a été faite en utilisant un cytomètre à flux Facstar + de la société Becton Dickinson.

### Résultats

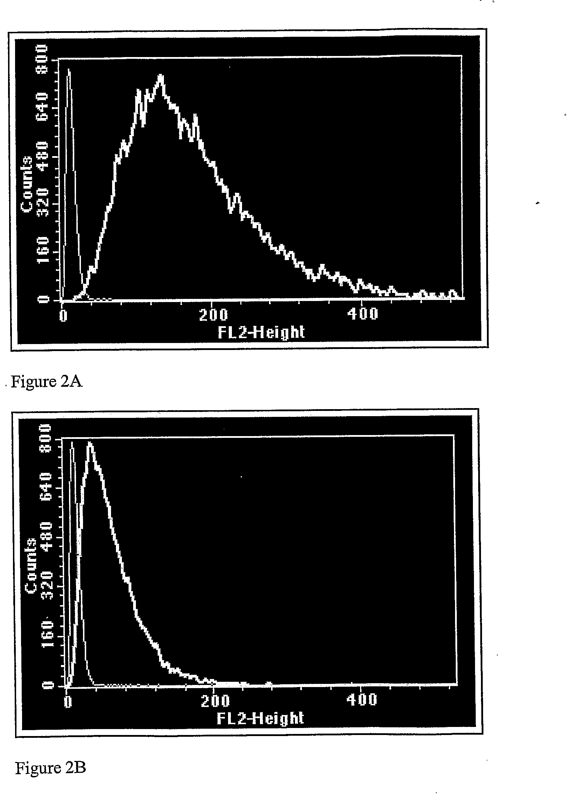

L'EC4b a conduit à observer un canal moyen de fluorescence de 271,5 alors que l'EC4d obtenu après traitement avec la trypsine a conduit à observer un canal moyen de fluorescence de 57,05 et les érythrocytes contrôles ont donné un canal moyen de fluorescence de 14,97 comme le montrent les figures 2A ou 2B respectivement.

Des courbes de calibration (figure 3) ont été obtenues avec des érythrocytes recouverts de C4d in vitro, traités ou non par la trypsine (ce qui conduit à deux concentrations de C4d différentes, utilisables pour déterminer la concentration en C4d d'échantillons pathologiques. Les courbes ont été établies à des moments différents ou dans des conditions différentes.
CMFC = corrected Mean Fluorescence Channel (bruit de fond soustrait) et AU = Arbitrary Units.

### REFERENCES

- 1.: Habicht, A. et al. A case of severe C4d-positive kidney allograft dysfunction in the absence of histomorphologie features of rejection. Wien Klin Wochenschr. 114. 945-948 (2002).
- 2.: Regele, H. et al. Capillary déposition of complément split product C4d in renal allografts is associated with basement membrane injury in peritubular and glomerular capillaries : a contribution of humoral immunity to chronic allograft rejection. J. Am Soc Nephrol. 13. 2371-2380 (2002).
- 3.: Magro, C.M. et al. Use of C4d as a diagnostic adjunct in lung allograft biopsies. Am J Transplant. 3, 1143-1154 (2003).
- 4.: Duong Van Huyen, J.P. et al. Acute vascular humoral rejection in a sensitized cardiac graft recipient: diagnostic value of C4d immunofluorescence. Hum Pathol. 35. 385-388 (2004).
- 5.: Miller. G.G. et al. Acute humoral rejection of human lung allografts and élévation of C4d in bronchoalveolar lavage fluid. Am J Transplant. 4. 1323-1330 (2004).
- 6.: Ionescu, D.N. et al. C4d déposition in lung allografts is associated with circulating anti-HLA alloantibody. Transpl Immunol. 15. 63-68 (2005)
- 7.: Weinstein, D. et al. Ultra-late antibody-mediated rejection 30 years after a living-related renal allograft. Am J Transplant. 5. 2576-2581 (2005).
- 8.: Manzi. S. et al. Measurement of erythrocyte C4d and complément receptor 1 in systemic lupus erythemalosus. Arthritis Rheum. 50. 3596-3604 (2004).
- 9.: Liu, C.C., Manzi, S. & Ahearn, J.M. Related biomarkers for systemic lupus erythematosus : a review and perspective. Curr Opin Rheumatol. 17, 543-549 (2005).
- 10.: Liu, C.C. et al. Reticulocytes bearing C4d as biomarkers of disease activity for systemic lupus erythematosus. Arthritis Rheum. 52. 3087-3099 (2005).
- 11.: Freysdottir, J. et al. A flow cytometric assay for measuring complément receptor 1 (CR1) and the complément fragments C3d and C4d on erythrocytes. J. Immunol Methods. 142. 45-52 (1991).
- 12.: Chakravarti, D.N. Campbell, R.D. & Porter, R.R. The chemical structure of the C4d fragment of the human complément component C4. Mol. Immunol. 11. 1187-1197 (1987).
- 13.: Atkinson, J.P. et al. Origin of the fourth component of complément related Chido and Rodgers blood group antigens. Complement. 5. 65-76 (1988).
- 14.: Tilley, C.A. Romans, D.G. & Crookston. M.C. Localisation of Chido and Rodgers déterminants to the C4d fragment of human C4. Nature. 276, 713-715 (1978).
- 15.: Kerr Anderson, W.II & Stroud, R.M. Génération and enrichment of C4d in whole sérum. J. Immunol Methods. 29. 323-330 (1979)
- 16.: Yu, C.Y. Bell, K.T. Giles, C.M., Campbell, R.D. & Porter. R.R. Structural basis of the polymorphism of human complement components C4A and C4B: gene size, reactivity and antigenicity. EMBO J. 5, 2873-2881 (1986).
- 17.: Spycher, M.O. et al. Human sérum induced opsonization of immunoglobulin bi-coated polystyrene microspheres with complement components C3 and C4 as measured by flow cytometry. J. Immunol Methods. 45. 83-92 (1991).
- 18.: Cohen. J. II et al, Enumeration of CR1 complement receptors on erythrocytes using a new method for detecting low density cell surface antigens by flow cytometry. J. immunol. Methods. 99. 53-58 (1987).
- 19.: Nadasdy. G.M. et al. Comparative study for the detection of peritubular capillary C4d déposition in human renal allografts using différent méthodologies. Hum Pathol 36, 1178-85 (2005).
- 20.: Racusen L.C. et al The Banff 97 working classification of renal allograft pathology Kidney Int.Feb:55(2):713-23 (1999).
- 21.: Racusen L.C. et al Antibody-mediated rejection criteria-an addition to the Banff 97 classification of the renal allograft rejection Am J Tranplant Jun 3(6): 708-14 (2003).

## Revendications

1. Procédé de détection *in vitro* du risque de rejet humoral de transplantation, chez un patient ayant reçu une transplantation d'organe, comprenant la détection quantitative, par cytométrie en flux, du composant du complément C4d déposé sur les érythrocytes contenus dans un échantillon de sang prélevé chez le patient, ledit procédé comprenant les étapes suivantes :
(i) quantification d'un standard interne constitué de suspension(s) stabilisée(s) d'érythrocytes recouverts d'une quantité connue de C4d, ledit C4d étant lié par liaison covalente directe ou indirecte aux érythrocytes et la quantité de C4d fixée sur les érythrocytes étant comprise entre 100 à 10000 molécules de C4d, et
(ii) quantification du C4d déposé sur les érythrocytes d'un échantillon de sang prélevé chez le patient avant la transplantation d'organe, puis sur un échantillon de sang prélevé chez ce patient après la transplantation d'organe.

2. Procédé de détection quantitative *in vitro* du C4d déposé sur les érythrocytes contenus dans un échantillon de sang prélevé chez un patient, comprenant les étapes suivantes :
a) d'une part le marquage, au moyen d'une première molécule associée à un marqueur ayant la capacité de reconnaître et de lier spécifiquement le C4d déposé sur les érythrocytes dudit échantillon de sang ; et d'autre part le marquage, au moyen d'une molécule associée à un marqueur, et ayant la capacité de reconnaître et de lier spécifiquement le C4d, d'une ou plusieurs compositions d'érythrocytes stabilisés recouverts d'une quantité connue de C4d, ledit C4d étant lié par liaison covalente directe ou indirecte aux érythrocytes et la quantité de C4d fixée sur les érythrocytes étant comprise entre 100 à 10000 molécules de C4d, ladite (lesdites) compositions constituant un standard interne pour la quantification;
b) l'introduction dans un cytomètre en flux, de l'échantillon à quantifier et de la composition de standard interne ;
c) la détection du signal induit par chacun des susdits marqueurs ;
d) la quantification du signal associé au C4d déposé sur les érythrocytes de l'échantillon testé et, optionnellement
e) une étape de comparaison de la valeur du signal de fluorescence obtenu pour l'échantillon, ou du nombre déduit de la quantité de C4d présent sur les érythrocytes, avec des valeurs de fluorescence ou de la quantité de C4d mesurés sur un échantillon de sang du patient prélevé avant la transplantation, ou des valeurs dites normales de quantité de C4d.

3. Procédé selon la revendication 2, dans lequel les mesures de fluorescences sont effectuées par analyse en cytométrie en flux, et lorsque les marqueurs sont des fluorochromes, la mesure de fluorescence est une mesure de l'intensité de fluorescence du canal moyen de fluorescence de chaque population d'érythrocytes considérés.

4. Procédé selon l'une quelconque des revendications 2 à 3, dans lequel la mesure de fluorescence est traduite en nombre de molécules de C4d données par une fluorescence équivalente à celle obtenue avec les érythrocytes marqués considérés.

5. Utilisation du procédé selon l'une quelconque des revendications 2 à 4, pour la détection *in vitro* du risque de rejet humoral de transplantation.

6. Procédé selon l'une quelconque des revendications 1 à 4, ou utilisation selon la revendication 5 dans lequel le C4d est détecté au moyen d'une molécule ayant la capacité de reconnaître et de lier spécifiquement ledit C4d, ladite molécule étant constituée par un anticorps ou un fragment d'anticorps conjugué à une molécule fluorescente, ou par un anticorps qui est un anticorps monoclonal anti-C4d ou un fragment d'un tel anticorps.

7. Utilisation d'un kit comprenant :
- une ou plusieurs compositions constituant un standard interne de quantification du C4d, ladite (lesdites) compositions étant constituée(s) d'une ou de suspension(s) stabilisée(s) d'érythrocytes recouverts d'une quantité connue de C4d, ledit C4d étant lié par liaison covalente directe ou indirecte aux érythrocytes et la quantité de C4d fixée sur les érythrocytes est comprise entre 100 à 10000 molécules de C4d,
- une courbe de calibration pour les compositions de standard interne, permettant de relier les mesures relatives d'intensité de fluorescence, à des unités arbitraires ou sites antigéniques C4d, ladite courbe étant obtenue par une analyse en cytométrie en flux, et le bruit de fond étant le cas échéant soustrait pour obtenir une valeur corrigée,
- le cas échéant, une composition de molécules ayant la capacité de reconnaître et de lier de façon spécifique le C4d, lesdites molécules étant de préférence marquées, par exemple par un fluorochrome, pour la détection d'un risque de rejet humoral de transplantation.

## Patentansprüche

1. Verfahren zum In vitro-Nachweis der Gefahr der humoralen Transplantatabstoßung bei einem Patienten, der eine Organtransplantation erhielt, welches den quantitativen Nachweis der auf den Erythrozyten abgelagerten Komplementkomponente C4d in einer beim Patienten entnommenen Blutprobe per Durchflusszytometrie umfasst, wobei besagtes Verfahren folgende Schritte umfasst:
(i) die Quantifizierung eines internen Standards bestehend aus einer oder mehreren stabilisierten Erythrozyten-Suspensionen, die mit einer bekannten Menge an C4d beschichtet sind, wobei besagtes C4d über eine kovalente direkte oder indirekte Bindung an die Erythrozyten gebunden ist und die an den Erythrozyten fixierte Menge an C4d zwischen 100 und 10000 C4d-Moleküle beträgt, und
(ii) die Quantifizierung des auf den Erythrozyten abgelagerten C4d einer beim Patienten vor der Organtransplantation entnommenen Blutprobe, und einer bei diesem Patienten nach der Organtransplantation entnommenen Blutprobe.

2. Verfahren zum quantitativen In vitro-Nachweis des auf den Erythrozyten abgelagerten C4d einer beim Patienten entnommenen Blutprobe, welches folgende Schritte umfasst:
a) zum einen das Markieren mittels eines mit einem Marker assoziierten ersten Moleküls, das die Fähigkeit hat, das auf den Erythrozyten der besagten Blutprobe abgelagerte C4d spezifisch zu erkennen und zu binden; und zum anderen das Markieren mittels eines mit einem Marker assoziierten Moleküls, das die Fähigkeit hat, das C4d einer oder mehrerer stabilisierter Erythrozyten-Zusammensetzungen, die mit einer bekannten Menge an C4d beschichtet sind, spezifisch zu erkennen und zu binden, wobei das C4d über eine kovalente direkte oder indirekte Bindung an die Erythrozyten gebunden ist und die Menge des auf den Erythrozyten fixierten C4d zwischen 100 und 10 000 C4d-Moleküle beträgt, wobei die Zusammensetzung oder die Zusammensetzungen einen internen QuantifizierungsStandard bilden;
b) die Einführung der zu quantifizierenden Probe und der internen Standardzusammensetzung in ein Durchflusszytometer;
c) den Nachweis des durch jeden der oben genannten Marker induzierten Signals;
d) die Quantisierung des Signals, das mit dem auf den Erythrozyten der getesteten Blutprobe abgelagerten C4d assoziiert ist, und optional
e) ein Schritt, in dem der Wert des für die Probe erhaltenen Fluoreszenzsignals oder die aus der Menge der auf den Erythrozyten vorhandenen C4d abgeleitete Anzahl verglichen wird mit den Fluoreszenzwerten oder der Menge an C4d, die in einer dem Patienten vor der Transplantation entnommenen Blutprobe gemessen wurden, oder mit den sogenannten normalen Mengenwerten für C4d.

3. Verfahren nach Anspruch 2, in dem die Fluoreszenzmessung durch eine Durchflusszytometrieanalyse erfolgt, und wenn die Maker fluorchromiert sind, die Fluoreszenzmessung eine Messung der Fluoreszenzintensität des mittleren Fluoreszenzkanals jeder berücksichtigten Erythrozytenpopulation ist.

4. Verfahren nach einem der Ansprüche 2 bis 3, in dem die Fluoreszenzmessung durch die Anzahl der C4d-Moleküle ausgedrückt wird, die bei einer Fluoreszenz entstehen, die der mit den berücksichtigten markierten Erythrozyten erzielten Fluoreszenz entspricht.

5. Benutzung des Verfahrens nach einem der Ansprüche 2 bis 4 zum In vitro-Nachweis der Gefahr der humoralen Transplantationsabstoßung.

6. Verfahren nach einem der Ansprüche 1 bis 4, oder Benutzung nach Anspruch 5, bei dem oder der das C4d mittels eines Moleküls nachgewiesen wird, das die Fähigkeit hat, besagtes C4d spezifisch zu erkennen und zu binden, wobei besagtes Molekül durch einen Antikörper oder ein Antikörperfragment konjugiert mit einem fluoreszierenden Molekül gebildet wird, oder durch einen Antiköper, der ein monoklonaler Anti-C4d-Antikörper oder ein Fragment eines solchen Antikörpers ist.

7. Benutzung eines Sets, welches folgendes umfasst:
- eine oder mehrere Zusammensetzungen, die einen internen Quantifizierungsstandard für C4d bilden, wobei besagte Zusammensetzung(en) aus einer oder mehreren stabilisierten Erythrozyten-Suspensionen gebildet werden, die mit einer bekannten Menge an C4d beschichtet sind, wobei besagtes C4d über eine kovalente direkte oder indirekte Bindung an die Erythrozyten gebunden ist und die Menge des an den Erythrozyten fixierten C4d zwischen 100 und 10000 C4d-Moleküle beträgt,
- eine Kalibrierungskurve für die Zusammensetzungen des internen Standards, die die Verknüpfung der relativen Messungen der Fluoreszenzintensität mit den willkürlichen Einheiten oder antigenen C4d-Stellen ermöglicht, wobei besagte Kurve durch eine Durchflusszytometrieanalyse erhalten wird und das Hintergrundrauschen gegebenenfalls subtrahiert wird, um einen berichtigten Wert zu erhalten,
- gegebenenfalls eine Zusammensetzung von Molekülen mit der Fähigkeit, das C4d auf spezifische Weise zu erkennen und zu binden, wobei besagte Moleküle vorzugsweise markiert sind, zum Beispiel durch ein Fluorchrom, um die Gefahr einer humoralen Transplantatabstoßung zu erkennen.

## Claims

1. Method of *in vitro* detection of the risk of humoral rejection of transplantation in a patient having received an organ transplant, comprising quantitative detection, by flow cytometry, of the complement component C4d deposited on the erythrocytes contained in blood sample collected from the patient, wherein said method comprises the following stages:
(i) quantification of an internal standard consisting of stabilised suspension (s) of erythrocytes covered with a known quantity of C4d, wherein said C4d is bound to the erythrocytes by direct or indirect covalent binding and the quantity of C4d bound to the erythrocytes is between 100 and 10,000 molecules of C4d, and
(ii) quantification of the C4d deposited on the erythrocytes of a blood sample collected from the patient before the organ transplant and subsequently on a blood sample collected from this patient after the organ transplant.

2. Method of *in vitro* detection of the C4d deposited on the erythrocytes contained in a blood sample collected from a patient, comprising the following stages:
a) on the one hand marking, by means of a first molecule associated with a marker capable of recognising and specifically binding the C4d deposited on the erythrocytes of said blood sample; and on the other hand, marking, by means of a molecule associated with a marker, capable of recognising and specifically binding the C4d, of one or more stabilised erythrocyte compositions covered with a known quantity of C4d, wherein said C4d is bound to the erythrocytes by direct or indirect covalent binding and the quantity of C4d bound to the erythrocytes is between 100 and 10,000 molecules of C4d, said composition(s) forming an internal standard for quantification;
b) introduction of the sample to be quantified and the internal standard composition into a flow cytometer;
c) detection of the signal induced by each of said markers;
d) quantification of the signal associated with the C4d deposited on the erythrocytes of the sample tested and, optionally
e) a stage of comparing the value of the fluorescence signal obtained for the sample, or of the deduced number of the quantity of C4d present on the erythrocytes, with values of fluorescence or of the quantity of C4d measured on a blood sample collected from the patient before the transplant, or of the so-called normal values for the quantity of C4d.

3. Method according to claim 2, wherein the fluorescence measurements are carried out by flow cytometric analysis, and when the markers are fluorochromes, the fluorescence measurement is a measurement of the fluorescence intensity of the mean fluorescence channel of each population of erythrocytes considered.

4. Method according to any one of claims 2 to 3, wherein the fluorescence measurement is translated into a number of C4d molecules yielded by a fluorescence equivalent to that obtained with the considered labelled erythrocytes.

5. Use of the method according to any one of claims 2 to 4, for *in vitro* detection of the risk of humoral rejection of transplantation.

6. Method according to any one of claims 1 to 4, or use according to claim 5, in which the C4d is detected by means of a molecule capable of recognising and specifically binding said C4d, wherein said molecule consists of an antibody or antibody fragment conjugated to a fluorescent molecule, or by an antibody which is an anti-c4D monoclonal antibody or a fragment of such an antibody.

7. Use of a kit comprising:
- one or several compositions forming an internal standard for quantification of the C4d, wherein said composition(s) consist(s) of a stabilised erythrocyte(s) suspension or suspensions covered with a known quantity of C4d, wherein said C4d is bound to the erythrocytes by direct or indirect covalent binding and the quantity of C4d bound to the erythrocytes is between 100 and 10,000 molecules of C4d,
- a calibration curve for the internal standard compositions, allowing linking of the relative fluorescence intensity measurements to arbitrary units or C4d antigen sites, wherein said curve is obtained by a flow cytometric analysis and the background noise is subtracted if necessary to obtain a corrected value,
- if necessary, a molecule composition capable of recognising and specifically binding the C4d, wherein said molecules are preferably labelled, for example by a fluorochrome, for detection of the risk of humoral rejection of transplantation.
